# EUROPEAN PATENT APPLICATION

(11) **EP 1 550 727 A1**
(43) Date of publication of application: **06.07.2005**
(21) Application number: 02777831.5
(22) Date of filing: 11.10.2002
(51) Int. Cl.: C12P 17/10, C07D 405/06, A61K 31/403, A61P 3/06, A61P 9/00, A61P 9/10, A61P 43/00

(54) **NOVEL MACROPHAGE FOAM CELL FORMATION INHIBITOR FKA-25 AND P ROCESS FOR PRODUCING THE SAME**

(71) Applicant: THE KITASATO INSTITUTE, Tokyo 108-8642 (JP)
(72) Inventor: OMURA, Satoshi, Setagaya-ku, Tokyo 157-0076 (JP); TOMODA, Hiroshi, Chofu-shi, Tokyo 182-0034 (JP); MASUMA, Rokuro, Minato-ku, Tokyo 108-0073 (JP)
(74) Representative: Duckworth, Timothy John
(86) International application number: PCT/JP2002/010585
(87) International publication number: WO 2004/033703

(57) **Abstract**

The present invention relates to a novel FKA-25 substance, which inhibits formation of the foam macrophage originated from mouse, having inhibitory action against formation of the foam macrophage and a process for production thereof. The process includes culturing Pseudobotrytis sp. FKA-25 belonging to genus Pseudobotrytis sp. and having ability to produce FKA-25 substance in a medium, accumulating FKA-25 substance in the cultured medium, and collecting FKA-25 substance from the cultured mass. The obtained FKA-25 substance specifically inhibits the formation of the foam macrophage originated from mouse and is expected to be useful for prevention and treatment of arteriosclerosis and causative diseases therefrom.

## Description

### Technical field

The present invention relates to novel FKA-25 substance having inhibitory action against formation of the foam macrophage and a process for production thereof. More particularly, the present invention pertains the FKA-25 substance (hereinafter sometimes designates as FKA-25) which inhibits specifically formation of the foam macrophage originated from mouse and is useful for prevention and treatment of various diseases such as arteriosclerosis, myocardial infarction, cerebral hemorrhage and cerebral apoplexy, and production thereof.

### Background art

Recently life-style related diseases such as hyperlipidemia and obesity of adults are increased due to changes of the eating habit. Such life-style related diseases have been known to develop atherosclerosis, further to the pathologic state directly connecting to death such as myocardial infarction, cerebral hemorrhage and cerebral apoplexy (Matsuzawa, Y., Nippon Rinsho, 59, 189-194, 2001) . At present, drugs, for example the statin series drug such as pravastatin, fluvastatin, cerivastatin and atorvastatin are used for prevention and treatment of atherosclerosis. These drugs exhibit reducing effect of blood cholesterol level due to inhibiting HMG-CoA reductase, a rate limiting enzyme of the cholesterol biosynthesis in vivo. However, arteriosclerosis is developed by complex mechanisms, and development of drugs having different mechanism of action is further requested.

It has been known that in the initial lesion of atherosclerosis, macrophage infiltrated into the arterial endothelium recognizes denatured LDL generated by any degeneration such as oxidation and glucosylation of low density lipoprotein (hereinafter sometimes designates as LDL) flowing in the blood stream, incorporates the denatured LDL indefinitely, and hydrolyses such the denatured LDL to generate free cholesterol and lipid acid, which are converted to cholesteryl ester and triacylglycerol and these are accumulated in the cytoplasm as lipid droplets. Then the macrophage is converted to the foam macrophage, as a result, atherosclerosis is progressed (Goldstein, J. L. et al. Proc. Natl. Acad. Sci. USA, 76, 333-337, 1979, and Gerrity, R. G. , Am. J. Pathol. 103, 181-190, 1981).

Consequently, a substance inhibiting macrophage foaming process is expected to suppress directly development of atherosclerotic lesion, however drugs having such effect including, for example, statin series drugs such as pravastatin, fluvastatin, cerivastatin and atorvastatin, have not been known.

### Disclosure of the invention

In such circumstances, it is expected that providing substance having inhibitory action against formation of the foam macrophage brings about good news to the human health as a new drug for directly acting in atherosclerotic lesion and suppressing its progress.

The present invention provides novel FKA-25 substance, which can satisfy such expectation, having inhibitory action against formation of the foam macrophage and production thereof. The present invention further provides drugs for prevention and treatment of arteriosclerosis, myocardial infarction, cerebral hemorrhage and cerebral apoplexy comprising FKA-25 having inhibitory action against formation of the foam macrophage as an active ingredient and a microorganism producing FKA-25 substance.

We have studied for solving such problems and further studied for finding novel substance having inhibitory action against formation of foam macrophage. We have further studied to continue isolating microorganisms from various soil samples and studied extensively microbial products, and found that the substance having inhibitory action against formation of form macrophage was produced in the cultured liquid of fungus FKA-25 strain newly isolated from soil sample. We have isolated and purified the substance having inhibitory action against formation of foam macrophage from the cultured mass and found the substance having chemical structure represented by the formula hereinbelow. Since the substance having such the chemical structure has not known before, the substance is designated as FKA-25 (or FKA-25 substance).

An object of the present invention is to provide novel FKA-25 substance represented by the following chemical formula, 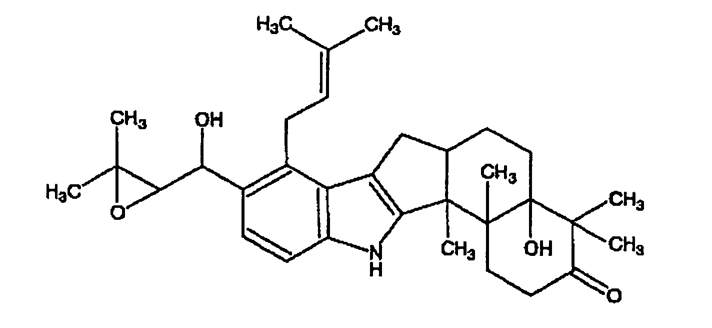 having inhibitory action against formation of the foam macrophage.

Another object of the present invention is to provide a process for production of novel FKA-25 substance having inhibitory action against formation of the foam macrophage comprising culturing a microorganism belonging to genus Pseudobotrytis having ability to produce FKA-25 substance, accumulating FKA-25 substance in the cultured mass and isolating FKA-25 substance therefrom.

Further object of the present invention is to provide a microorganism wherein the microorganism belonging to genus Pseudobotrytis is Pseudobotrytis sp. FKA-25.

Still another object of the present invention is to provide the novel FKA-25 substance having inhibitory action against formation of the foam macrophage used for prevention or treatment of diseases caused by process accumulating lipid droplets of cholesteryl ester and triacylglycerol converted from cholesterol and fatty acid in the cytoplasm and forming the foam cell.

Still more another object of the present invention is to provide the novel FKA-25 substance having inhibitory action against formation of the foam macrophage used for prevention or treatment of arteriosclerosis caused by process accumulating lipid droplets of cholesteryl ester and triacylglycerol converted from cholesterol and fatty acid in the cytoplasm and forming the foam cell.

More further object of the present invention is to provide the novel FKA-25 substance having inhibitory action against formation of the foam macrophage used for prevention or treatment of myocardial infarction caused by process accumulating lipid droplets of cholesteryl ester and triacylglycerol converted from cholesterol and fatty acid in the cytoplasm and forming the foam cell.

Still more further object of the present invention is to provide the novel FKA-25 substance having inhibitory action against formation of the foam macrophage used for prevention or treatment of cerebral hemorrhage caused by process accumulating lipid droplets of cholesteryl ester and triacylglycerol converted from cholesterol and fatty acid in the cytoplasm and forming the foam cell.

Another more further object of the present invention is to provide use of the novel FKA-25 substance having inhibitory action against formation of the foam macrophage used for prevention or treatment of cerebral apoplexy caused by process accumulating lipid droplets of cholesteryl ester and triacylglycerol converted from cholesterol and fatty acid in the cytoplasm and forming the foam cell.

Further object of the present invention is to provide use of the novel FKA-25 substance having inhibitory action against formation of the foam macrophage for production of drug for prevention or treatment of arteriosclerosis caused by process accumulating lipid droplets of cholesteryl ester and triacylglycerol converted from cholesterol and fatty acid in the cytoplasm and forming the foam cell.

Further object of the present invention is to provide use of the novel FKA-25 substance having inhibitory action against formation of the foam macrophage for drug for prevention or treatment of myocardial infarction caused by process accumulating lipid droplets of cholesteryl ester and triacylglycerol converted from cholesterol and fatty acid in the cytoplasm and forming the foam cell.

Further object of the present invention is to provide use of the novel FKA-25 substance having inhibitory action against formation of the foam macrophage for drug for prevention or treatment of cerebral hemorrhage caused by process accumulating lipid droplets of cholesteryl ester and triacylglycerol converted from cholesterol and fatty acid in the cytoplasm and forming the foam cell.

Further object of the present invention is to provide use of the novel FKA-25 substance having inhibitory action against formation of the foam macrophage for drug for prevention or treatment of cerebral apoplexy caused by process accumulating lipid droplets of cholesteryl ester and triacylglycerol converted from cholesterol and fatty acid in the cytoplasm and forming the foam cell.

The microorganism having ability to produce FKA-25 substance of the present invention represented by the formula hereinbefore (hereinafter designates as FKA-25 substance producing microorganism) belongs genus Pseudobotrytis, and, for example, a strain Pseudobotrytis sp. FKA-25, which was newly isolated from the soil sample of Yakushima, Kagoshima Pref., Japan, by the present inventors), is the most effectively used strain in the present invention.

Taxonomical properties of the strain Pseudobotrytis sp. FKA-25 of the present invention are exemplified as follows.

### (I) Morphological properties

The strain is grown moderately on potato dextrose agar, corn meal agar, Miura's agar and malt extract agar. Bearing of conidia is good on potato dextrose agar and malt extract agar, and is slightly suppressive on corn meal agar and Miura' s agar.

Microscopic observation of colonies grown on Miura' s agar shows: hyphae have septa, and conidiophore is erecting from basal mycelium with sometimes branching from aerial mycelium. Length: 180 - 390 µm × 4.5 - 6.0 µm, colored surface, near the apex is colorless with smooth surface. Apical part is slightly bulged with 6 - 14 verticillations of conidia forming structures (20 - 30 µm × 2. 3 - 3.75 µm). Apices of conidiogenous cells are bulged with abundantly forming conidia from penumbral odontogenic spikes. Sympodioconidia formed from the odontogenic spikes are ellipsoidal to clavate, didymospore with slightly necked septal wall, having papillate cleavage trace in the basal region, size 8.0 - 10.5 µm × 3.0 - 4.5 µm, pale dark brown. Chlamydospore is pale brown to dark brown, globose to subglobose (5.5 - 8.5 µm) or inverted obovate (8.5 - 12.0 µm × 7.0 - 8.0 µm).

### (II) Culture properties on various media

Results of macroscopic observation on various agar media cultured at 25°C for 14 days are shown as follows.

| | | | | |
|---|---|---|---|---|
| Medium | Growth condition on medium (diameter of colony) | Color tone of colony surface | Color tone of colony reverse | Soluble pigment |
| Potato dextrose agar | Moderate (49 - 53 mm) Velvety - powdery Slightly irregular edge | Dark olive - dull grayish brown | Dark grayish brown - dark yellowish brown | None |
| Cornmeal agar | Moderate (40 - 43 mm) Glabrous - powdery, linearly Irregular edge | Dark olive - dull yellowish brown | Olive - dull yellowish brown | None |
| Mirura's agar | Moderate (36 - 38 mm) Glabrous - powdery, linearly | Dark olive | Dark olive | None |
| | Irregular edge | | | |
| Malt extract agar | Moderate (50 - 51 mm) Floccose - velvety Smooth edge | Pale yellowish green - pale gray | Pale dark brown | None |

### (III) Physiological properties

### 1) Optimum growth condition

Optimum growth condition of the strain is pH 4 - 6 at 25.5 - 34.0°C.

### 2) Growth range

Growth range of the strain is pH 3 - 7 at 12.5 - 39.5°C.

### 3) Nature

### Aerobic

As a result of comparison with the present strain exhibiting above morphological properties, culture properties and physiological properties and known microorganisms, the strain was identified as the strain belonging to genus Pseudobotrytis. The strain was referred to Pseudobotrytis sp. FKA-25 and was deposited in International Patent Organism Depositary, National Institute of Advanced Industrial Science and Technology, AIST Tsukuba Central 6, 1-1, Higashi 1-Chome Tsukuba-shi, Ibaraki-ken, 305-8566 Japan according to Budapest Treaty on the International Recognition of the Deposit of Microorganisms for the Purposes of Patent Procedure on September 27, 2002 and received the deposition No. FERM BP-8199.

As for FKA-25 substance producing microorganism strain used in the present invention, the strain Pseudobotrytis sp. FKA-25 hereinbefore can be mentioned as a preferable example. However, it is well known that the microorganism is very easily mutated in the general mycological and can not be maintained constant in the mycological properties, and is mutated by natural means or artificial means, for example commonly used ultraviolet irradiation or mutation inducer such as N-methyl-N'-nitro-N-nitrosoguanidine and ethyl methansulfonate. Consequently, the strains belonging to genus Pseudobotrytis and having producing ability of FKA-25 substance represented by the chemical formula hereinbefore, including artificial mutants and natural mutants, can be used all in the present invention.

The production of FKA-25 substance of the present invention can be performed at first by culturing FKA-25 substance producing microorganism belonging to genus Pseudobotrytis. As for nutrient sources preferable for production of FKA-25 substance of the present invention, carbon sources which can be assimilable by microorganism, nitrogen sources which can be digestible, and if necessary nutrient medium containing inorganic salt, vitamin, etc. can be used. Examples of carbon sources are sugars such as glucose, fructose, maltose, lactose, galactose, dextrin and starch, and vegetable oil such as soybean oil. These are used alone or in combination.

Examples of nitrogen sources are peptone, yeast extract, meat extract, soybean powder, cotton seed powder, corn steep liquor, malt extract, casein, amino acids, urea, ammonium salts and nitrate. These are used alone or in combination. If necessary, salts such as phosphate, magnesium salt, calcium salt, sodium salt and potassium salt, heavy metal salt such as iron salt, manganese salt, copper salt, cobalt salt and zinc salt, vitamins and substances preferable for FKA-25 substance production can be added.

In the culture, when forming occurs, if necessary, antifoaming agent such as liquid paraffin, animal oil, vegetable oil, silicone oil and surface active agent can be added. The culture can be performed by liquid culture or solid culture, if above nutrient sources are contained. In general, the liquid medium is used for the culture. In case of small culture, the culture using flask is preferable.

In the large scale production using tank culture, in order to prevent growth delay of the microorganism in the production process, it is preferable that the production strain is at first inoculated and cultured in the relatively small amount of medium, and the cultured mass is transferred into the large tank and is continued to culture. In this case, composition of the medium used in the pre-cultivation and the medium used in the production culture can be same or different.

When the culture is performed in aeration with stirring, known method such as stirring by propeller and other mechanical stirring, rotary or shaking the fermenter, pumping or bubbling aeration can be applied. Sterilized air is used for aeration. Culturing temperature can be changed within ranges for production of FKA-25 substance by the FKA-25 substance producing strain, generally at 25 - 32°C, preferably around at 27°C. Culturing pH is generally 5 - 8, preferable around 7. Culturing time depends on the culturing condition and is generally about 7 days.

FKA-25 substance accumulated in the thus obtained cultured mass is generally found in the cultured mycelia. In order to collect FKA-25 substance from the cultured mycelia, means for collecting metabolites from common microorganism culture can be used alone or in combination, or repeatedly. For example, means such as filtration, centrifugation, dialysis, concentration, drying, freezing, adsorption, desorption, a method for applying solubility difference for various solvents (e.g. precipitation, crystallization, recrystallization, solvent extraction and counter current distribution) and chromatography can be used.

FKA-25 substance can be isolated and collected from the mycelial extraction. For example, the substance can be extracted from mycelia using organic solvent such as acetone, ethanol and methanol. After concentration of the extract, extraction using organic solvent such as chloroform and ethyl acetate can be performed. FKA-25 substance can be isolated by silica gel chromatography, Sephadex LH-20, ODS column chromatography, etc. after concentration of the extract.

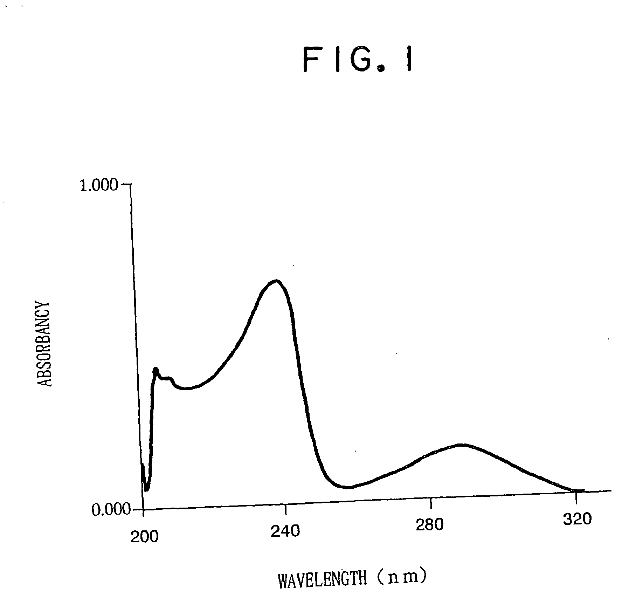

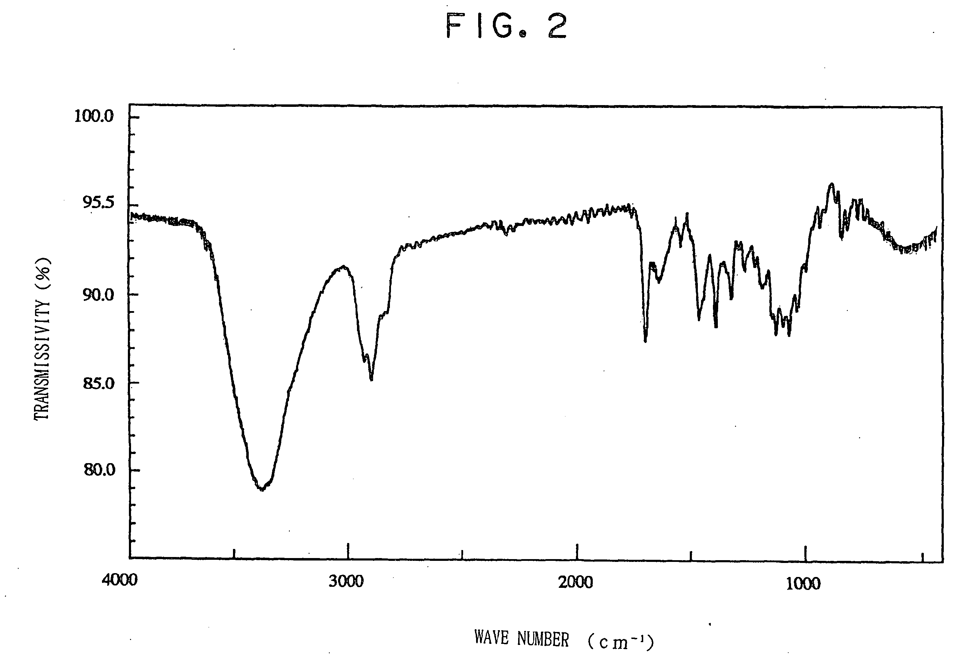

Physico-chemical properties of FKA-25 substance of the present invention are as follows.
(1) Nature: White powder
(2) Molecular weight: 519 (M+, electron bombardment mass spectroscopy)
(3) Molecular formula: C₃₃H₄₅O₄N
(4) Specific rotation: [α]_{D} = -18.0° (c = 0.1, in methanol)
(5) UV absorption spectrum (in methanol): Fig. 1, maximum absorption at 239 nm and 288 nm
(6) IR absorption spectrum (KBr tablet): Fig. 2, specific absorption bands at 3440.4, 3426, 2929, 1693, 1456 and 1378 cm⁻¹
(7) Solubility in solvents: Soluble in methanol, acetone, chloroform and ethyl acetate; slightly soluble in water and hexane
(8) Color reaction: Positive in sulfuric acid and Dragendorff; negative in ninhydrin
(9) ¹H-proton NMR spectrum (in deuterated chloroform) was measured by using XL-400 (Varian Inc. Japan). Chemical shifts (ppm) are:
   7.88(1H), 7.21(2H), 5.17(1H), 4.81(1H), 3.75(1H), 3.65(1H), 3.27(1H), 2.86(1H), 2.68(2H), 2.54(1H), 2.00(2H), 1.82(1H), 1.78(3H), 1.72(1H), 1.64(3H), 1.63(2H), 1.62(1H), 1.30(3H), 1.32(3H), 1.29(3H), 1.25(3H), 1.24(3H), 1.11(3H)
   (H: proton number)
(10) ¹³C-NMR spectrum (in deuterated chloroform) was measured by using XL-400 (Varian Inc. Japan). Chemical shifts (ppm) are: 216.9s, 152.8s, 139.7s, 131.4s, 131.3s, 129.8s, 125.6s, 124.9d, 120.1d, 117.2s, 110.2d, 81.4s, 69.6d, 68.2d, 60.1s, 54.8s, 52.9s, 49.5d, 43.6s, 34.6t, 32.4t, 29.8t, 29.5t, 29.0t, 25.8q, 25.1q, 24.2q, 23.1q, 22.7q, 21.1q, 19.5q, 18.5q, 17.9q
   (s: singlet, d: doublet, t: triplet and q: quartet)
(11) Differentiation from acidity, neutrality and basicity: weak basic substance

Examining the above data of various physico-chemical properties and spectral data, FKA-25 substance was determined as having chemical structure represented by the following formula.

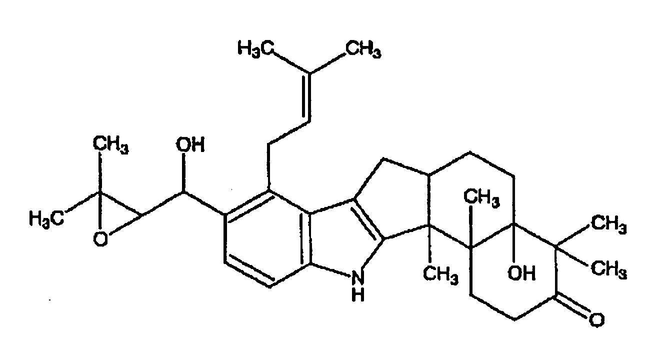

Inhibitory action of FKA-25 substance of the present invention against intraperitoneal formation of cholesteryl ester and lipid droplet in mice is explained as follows.

Formation of cholesteryl ester and lipid droplet in mice in the intraperitoneal macrophage was examined according to the method of Namatame et al. (J. Biochem. 125, 319-327, 1999).

Macrophages isolated from mice peritoneum were suspended in Dulbecco modified Eagle's medium containing 6.8% lipoprotein deficient serum (6.8% LPDS-DMEM) 2.0 × 10⁶ cells/ml, and plated each 0.25 ml into 48 well microplate (Corning Inc.) or slide chamber (Nunc Inc.).

The plate was incubated in 5% carbon dioxide incubator at 37°C for 2 hours, then cells without attachment were removed by washing with Hank's solution. After washing, cells were incubated with 6.8% LPDS-DMEM for 1 hour, and FKA-25 substance (2.5 µl methanol), liposome (a composition consisting of phosphatidylcholine / phosphatidylserine / dicetylphosphate / cholesterol = 10 : 10 : 2 : 15 (n mol) in 0.3 M glucose 10 µl) and [1-¹⁴C]oleic acid (5 µl, 0.05 µCi, 1 nmol) were added, then incubated for 14 hours.

Cultured supernatant was removed off and intracellular neutral lipid was extracted twice by adding hexane 0.6 ml and isopropanol 0.4 ml. The extract was concentrated, spotted on TLC plate (silica gel plate, the U.S., Merck Inc., thickness 0.5 mm), and developed with the solvent of hexane/diethyl ether/acetic acid (70 : 30 : 1, v/v). The separated [¹⁴C]cholesteryl oleate and [¹⁴C]triacylglycerol were quantitatively measured by using radio scanner (Ambis Inc., the U.S.). As a result, FKA-25 substance inhibited relatively and selectively the formation of [¹⁴C]cholesteryl oleate, and IC₅₀ of FKA-25 substance was determined as 4.0 µM, and FKA-25 substance inhibited the formation of triacylglycerol about 50% at 3.2 µM.

### Brief explanation of drawing

Fig 1 shows UV spectrum of FKA-25 substance of the present invention (in methanol).
Fig 2 shows IR spectrum of FKA-25 substance of the present invention (KBr tablet).

### Best mode carrying out the invention

The present invention is explained by mentioning example, but is not limited within the example.

Liquid medium (pH 6.0), each 100 ml, consisting of glucose 2.0%, yeast extract (Oriental Yeast Co., Japan) 0.2%, magnesium sulfate 7H₂O 0.05%, polypeptone 0.5%, potassium bisphosphate 0.1% and agar 0.1%, was added in the 500 ml Erlenmeyer flask, sterilized at 121°C for 15 minutes. One loopful of the strain of Pseudobotrytis sp. FKA-25 FERM BP-8199 cultured on the agar slant containing soluble starch 1.5%, yeast extract (Oriental Yeast Co., Japan) 0.4%, magnesium sulfate 7H₂O 0.05%, dipotassium phosphate 0.1% and agar 2.0% at 27°C, was inoculated and cultured at 27°C for 3 days using rotary shaker to obtain seed culture liquid.

Liquid medium (pH 6) 20 lit. consisting of saccharose 2.0%, glucose 1.0%, corn steep powder (Iwaki Co., Japan) 0.5%, meat extract (Kyokuto Seiyaku Co., Japan) 0.5%, calcium carbonate 0.3%, magnesium sulfate 0.05%, monopotassium phosphate 0.1% and agar 0.1%, was poured into a 30 lit. jar-fermenter and sterilized at 121°C for 60 minutes. Seed culture liquid 2 flasks was inoculated thereto and cultured at 250 rpm, aeration 15 lit. /min. at 27°C for 4 days.

Acetone 20 lit. was added to the cultured liquid and stirred for 30 minutes to obtain supernatant after centrifugation. The obtained acetone solution was concentrated in vacuo to obtain aqueous solution. Ethyl acetate 20 lit. was added to the obtained aqueous solution, stirred and centrifuged using Sharples centrifuge (10,000 rpm) to separate into aqueous layer and ethyl acetate layer. Anhydrous sodium sulfate 500 g was added to the obtained ethyl acetate layer, dehydrated and concentrated the ethyl acetate layer in vacuo to obtain crude substance I, 4.6 g. The crude substance was dissolved in a small amount of chloroform, mounted on the top of a silica gel column (35 g, the U. S. , Merck Inc. , silica gel 60, mesh 70-230 µm) packed with chloroform, washed the column with chloroform and eluted the active substance by using chloroform - methanol (9 : 1).

The eluted active substance was concentrated in vacuo to obtain crude substance II, 795 mg. The crude substance II was again dissolved in chloroform, mounted on the top of a silica gel column (17 g, the U.S., Merck Inc., silica gel 60, mesh 230-400 µm) packed with chloroform, washed the column with chloroform and eluted with chloroform - methanol (10 : 1) to obtain crude substance III.

The crude substance III was dissolved in a small amount of methanol, and the solution was treated with HPLC (PEGASIL ODS, ϕ20 × 250 mm, Senshu Kagaku Co., Japan). Absorption at 240 nm was detected by using 60% acetonitrile as a mobile phase, and the peak eluted at 16 minutes in the flow rate at 8 ml/min. was collected. The collected solution was concentrated in vacuo to obtain FKA-25 substance having inhibitory action against formation of the foam macrophage, 13.9 mg as the white powder.

### Industrial applicability

As explained hereinabove, FKA-25 substance obtained by culturing the strain of Pseudobotrytis sp. FKA-25 belonging to genus Pseudobotrytis having ability to produce FKA-25 substance, accumulating FKA-25 substance in the cultured mass and collecting FKA-25 substance from the cultured mass specifically inhibits the formation of the foam macrophage originated from mouse and is expected to be useful for prevention and treatment of arteriosclerosis and causative diseases therefrom.

## Claims

1. A novel FKA-25 substance represented by the following chemical formula, 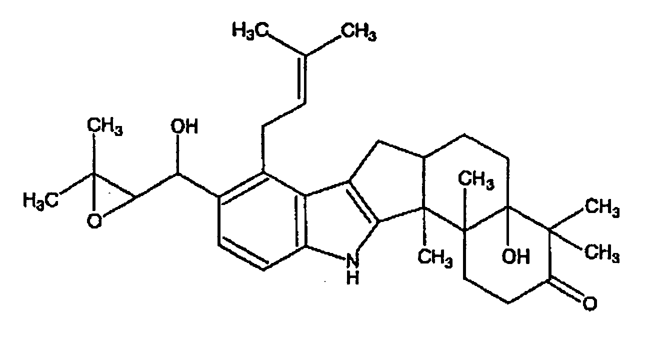 having inhibitory action against formation of the foam macrophage.

2. A process for production of novel FKA-25 substance having inhibitory action against formation of the foam macrophage comprising culturing a microorganism belonging to genus Pseudobotrytis having ability to produce FKA-25 substance, accumulating FKA-25 substance in the cultured mass and isolating FKA-25 substance therefrom.

3. The process for production of novel FKA-25 substance having inhibitory action against formation of the foam macrophage according to claim 2 wherein the microorganism having ability to produce FKA-25 substance is Pseudobotrytis sp. FKA-25 FERM BP-8199 or mutant thereof.

4. A microorganism belonging to genus Pseudobotrytis having ability to produce the substance according to claim 1.

5. The microorganism according to claim 4 wherein the microorganism having ability to produce FKA-25 substance is Pseudobotrytis sp. FKA-25 FERM BP-8199 or mutant thereof.

6. The novel FKA-25 substance having inhibitory action against formation of the foam macrophage according to claim 1 comprising using for prevention or treatment of diseases caused by process accumulating lipid droplets of cholesteryl ester and triacylglycerol converted from cholesterol and fatty acid in the cytoplasm and forming the foam cell.

7. The novel FKA-25 substance having inhibitory action against formation of the foam macrophage according to claim 1 comprising using for prevention or treatment of arteriosclerosis caused by process accumulating lipid droplets of cholesteryl ester and triacylglycerol converted from cholesterol and fatty acid in the cytoplasm and forming the foam cell.

8. The novel FKA-25 substance having inhibitory action against formation of the foam macrophage according to claim 1 comprising using for prevention or treatment of myocardial infarction caused by process accumulating lipid droplets of cholesteryl ester and triacylglycerol converted from cholesterol and fatty acid in the cytoplasm and forming the foam cell.

9. The novel FKA-25 substance having inhibitory action against formation of the foam macrophage according to claim 1 comprising using for prevention or treatment of cerebral hemorrhage caused by process accumulating lipid droplets of cholesteryl ester and triacylglycerol converted from cholesterol and fatty acid in the cytoplasm and forming the foam cell.

10. Use of the novel FKA-25 substance having inhibitory action against formation of the foam macrophage described in claim 1 comprising using for prevention or treatment of cerebral apoplexy caused by process accumulating lipid droplets of cholesteryl ester and triacylglycerol converted from cholesterol and fatty acid in the cytoplasm and forming the foam cell.

11. Use of the novel FKA-25 substance having inhibitory action against formation of the foam macrophage described in claim 1 for production of drug for prevention or treatment of arteriosclerosis caused by process accumulating lipid droplets of cholesteryl ester and triacylglycerol converted from cholesterol and fatty acid in the cytoplasm and forming the foam cell.

12. Use of the novel FKA-25 substance having inhibitory action against formation of the foam macrophage described in claim 1 for drug for prevention or treatment of myocardial infarction caused by process accumulating lipid droplets of cholesteryl ester and triacylglycerol converted from cholesterol and fatty acid in the cytoplasm and forming the foam cell.

13. Use of the novel FKA-25 substance having inhibitory action against formation of the foam macrophage described in claim 1 for drug for prevention or treatment of cerebral hemorrhage caused by process accumulating lipid droplets of cholesteryl ester and triacylglycerol converted from cholesterol and fatty acid in the cytoplasm and forming the foam cell.

14. Use of the novel FKA-25 substance having inhibitory action against formation of the foam macrophage described in claim 1 for drug for prevention or treatment of cerebral apoplexy caused by process accumulating lipid droplets of cholesteryl ester and triacylglycerol converted from cholesterol and fatty acid in the cytoplasm and forming the foam cell.
